# EUROPÄISCHE PATENTANMELDUNG

(11) **EP 1 702 606 A2**
(43) Veröffentlichungstag der Anmeldung: **20.09.2006**
(21) Anmeldenummer: 05023928.4
(22) Anmeldetag: 03.11.2005
(51) Int. Cl.: A61K 8/18

(54) **Ölhaltige Tensidgele**

(30) Priorität: 12.11.2004 DE 102004054842
(71) Anmelder: Cognis IP Management GmbH, 40589 Düsseldorf (DE)
(72) Erfinder: Müller, Michael, 40789 Monheim (DE)

(57) **Zusammenfassung**

Gegenstand der vorliegenden Patentanmeldung sind gelförmige kosmetische Zubereitungen enthaltend
(a) 5 bis 50 Gew.% Tenside,
(b) 5 bis 50 Gew.% Öle und/oder Wachse,
(c) 0 bis 15 Gew.% wasserlösliche Polyole,
   wobei die Komponente (a) mindestens 10 Gew.% in bezug auf die Gesamtzubereitung eines Citronensäureesters alkoxylierter Alkohole enthält und die Summe der Komponenten (a) und (b) in der Gesamtzubereitung 10 bis 70 Gew.% ausmacht.

## Beschreibung

### Gebiet der Erfindung

Die Erfindung befindet sich auf dem Gebiet der tensidhaltigen Gele, die als kosmetische Zubereitungen Verwendung finden.

### Stand der Technik

Üblicherweise zeigen wässrige Tensidformulierung ein stark eingeschränktes Aufnahmevermögen für pflegende Stoffen wie Öle und Wachse. Soll die Transparenz und die thermodynamische Stabilität der Tensidformulierung beibehalten werden, lassen sich bezogen auf die Tensideinwaage nur geringe Mengen an Ölen oder Wachsen einarbeiten. Eine Ausnahme bilden Mikroemulsionen, welche z.T. die Einarbeitung von Ölen zu über 100 Gew-% bezogen auf den Tensidanteil erlauben. Mikroemulsionen sind jedoch niedrigviskos und ihre Stabilitäten zeigen je nach verwendeten Tensidtyp eine ausgeprägte Temperaturabhängigkeit. Die niedrige Viskosität der Mikroemulsionen schränkt den Anwendungsbereich im kosmetischen Bereich stark ein, da für Griff und Auftragung des kosmetischen Produktes auf dem Körper eine erhöhte Viskosität notwendig ist.

Auch Einsatz der üblichen Verdickungsmittel ist mit Nachteilen behaftet, da sich diese auf Haut und Haaren ablagern können und die verdickten Zubereitungen häufig nicht die gewünschten Stabilitäten aufweisen.

Die Aufgabe der vorliegenden Erfindung hat somit darin bestanden, kosmetische tensidhaltige Gel-Formulierungen bereitzustellen, die Öle und/oder Wachse enthalten und möglichst ohne Verwendung von Verdickungsmitteln stabil sind. Die Gele sollen über eine ausgeprägte Formstabilität verfügen und von transparentem bis leicht trüben Aussehen sein. Weiterhin sollen sie ein sehr gutes Hautgefühl aufweisen.

### Beschreibung der Erfindung

Gegenstand der vorliegenden Erfindung sind gelförmige kosmetische Zubereitungen enthaltend
(a) 5 bis 50 Gew.% Tenside,
(b) 5 bis 50 Gew.% Öle und/oder Wachse,
(c) 0 bis 15 Gew.% wasserlösliche Polyole,
   wobei die Komponente (a) mindestens 10 Gew.% in bezug auf die Gesamtzubereitung eines Citronensäureesters alkoxylierter Alkohole enthält und die Summe der Komponenten (a) und (b) in der Gesamtzubereitung 10 bis 70 Gew.% ausmacht.
   Überraschenderweise wurde gefunden, daß mit Hilfe von Citronensäureestern alkoxylierter Alkohole als Basistensid Tensidformulierungen von hoher bis sehr hoher Viskosität erhalten werden, die bis zu 300 Gew% Öle oder Wachse bezogen auf den Tensidanteil enthalten können. Bei den extrem hochviskosen Formulierungen handelt es sich um Gele, die eine ausgeprägte Formstabilität aufweisen und dabei transparent bis leicht trüb sind. Dabei sind die erfindungsgemäßen gelförmigen Zubereitungen frei von polymeren Verdickem.

### Komponente (a)

Die erfindungsgemäßen gelförmigen Zubereitungen können als neben den Citronensäureestern alkoxylierter Alkohole weitere Tenside beinhalten. In einer besonderen Ausführungsform enthalten die erfindungsgemäßen Zubereitungen als Komponente (a) neben Citronensäureestern alkoxylierter Alkohole Cotenside, die ausgewählt sind aus der Gruppe, die gebildet wird von anionischen, kationischen, nichtionischen, amphoteren und/oder zwitterionischen Tensiden.

### Anionische Tenside

Typische Beispiele für anionische Tenside sind Seifen, Alkylbenzolsulfonate, Alkansulfonate, Olefinsulfonate, α-Methylestersulfonate, Sulfofettsäuren, Alkylsulfate, Alkylethersulfate, Mono- und Dialkylsulfosuccinate, Mono- und Dialkylsulfosuccinamate, Sulfotriglyceride, Monoglyceridsulfate, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate und Proteinfettsäurekondensate (insbesondere pflanzliche Produkte auf Weizenbasis).

### Kationische Tenside

Typische Beispiele für kationische Tenside sind quartäre Ammoniumverbindungen und Esterquats, insbesondere quaternierte Fettsäuretrialkanolaminestersalze.

### Nichtionische Tenside

Typische Beispiele für nichtionische Tenside sind Alk(en)yloligoglykoside, Fettsäure-N-alkylglucamide, Polyolfettsäureester, Zuckerester, Sorbitanester, Polysorbate, Alkoholethoxylate und Aminoxide. Vorzugsweise werden Alkyl- und/oder Alkenyloligoglucoside, die insbesondere der Formel (II) folgen,

**R**^{**2**}**O-[G]**_{**p**} **(II)**

in der R2 für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel **(II)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,5 liegt.

Der Alkyl- bzw. Alkenylrest R² kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R² kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Alkoholethoxylate werden herstellungsbedingt als Fettalkohol- oder Oxoalkoholethoxylate bezeichnet und folgen vorzugsweise der Formel **(III),**

**R**^{**1**}**O(CH**_{**2**}**CH**_{**2**}**O)**_{**n**}**H** (III)

in der R¹ für einen linearen oder verzweigten Alkyl- und/oder Alkenylrest mit 6 bis 22 Kohlenstoff-atomen und n für Zahlen von 1 bis 50 steht. Typische Beispiele sind die Addukte von durchschnittlich 1 bis 50, vorzugsweise 5 bis 40 und insbesondere 10 bis 25 Mol an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dime-risierung von ungesättigten Fettalkoholen anfallen. Bevorzugt sind Addukte von 10 bis 40 Mol Ethylenoxid an technische Fettalkohole mit 12 bis 18 Kohlenstoffatomen, wie beispielsweise Kokos-, Palm-, Palmkern- oder Talgfettalkohol.

### Zwitterionische und amphotere Tenside

Beispiele für geeignete amphotere bzw. zwitterionische Tenside sind Alkylbetaine, Alkylamidobetaine, Aminopropionate, Aminoglycinate, Imidazoliniumbetaine und Sulfobetaine. Beispiele für geeignete Alkylbetaine stellen die Carboxyalkylierungsprodukte von sekundären und insbesondere tertiären Aminen dar, die der Formel (IV) folgen, 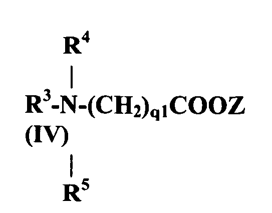 in der R3 für Alkyl- und/oder Alkenylreste mit 6 bis 22 Kohlenstoffatomen, R⁴ für Wasserstoff oder Alkylreste mit 1 bis 4 Kohlenstoffatomen, R⁵ für Alkylreste mit 1 bis 4 Kohlenstoffatomen, q1 für Zahlen von 1 bis 6 und Z für ein Alkali- und/oder Erdalkalimetall oder Ammonium steht. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische.

Weiterhin kommen auch Carboxyalkylierungsprodukte von Amidoaminen in Betracht, die der Formel (V) folgen, 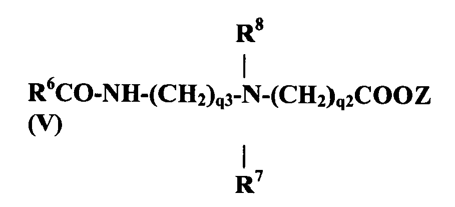 in der R⁶CO für einen aliphatischen Acylrest mit 6 bis 22 Kohlenstoffatomen und 0 oder 1 bis 3 Doppelbindungen, R⁷ für Wasserstoff oder Alkylreste mit 1 bis 4 Kohlenstoffatomen, R⁸ für Alkylreste mit 1 bis 4 Kohlenstoffatomen, q2 für Zahlen von 1 bis 6, q für Zahlen von 1 bis 3 und Z wieder für ein Alkali- und/oder Erdalkalimetall oder Ammonium steht. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von C_{8/18}-Kokosfettsäure-N,N-dime-thylaminopropylamid mit Natriumchloracetat.

Weiterhin kommen auch Imidazoliniumbetaine in Betracht. Auch bei diesen Substanzen handelt es sich um bekannte Stoffe, die beispielsweise durch cyclisierende Kondensation von 1 oder 2 Mol Fettsäure mit mehrwertigen Aminen wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der oben genannten Fettsäuren mit AEEA, vorzugsweise Imidazoline auf Basis von Laurinsäure oder wiederum C_{12/14-}Kokosfettsäure, die anschließend mit Natriumchloracetat betainisiert werden.

Im Sinne der vorliegenden Erfindung besonders bevorzugt sind dabei Zubereitungen, deren Komponente (a) neben Citronensäureestern alkoxylierter Alkohole Cotenside enthält, die ausgewählt sind aus der Gruppe, die gebildet wird von Betainen, acylierten Aminosäuren, Alkylethersulfaten, Alkylsulfaten und Alkyloligoglykosiden.

### Citronensäureestermischungen alkoxylierter Alkohole

Als Basistensid im Rahmen der Komponente (a) werden Citronensäureestermischungen alkoxylierter Alkohole verwendet. Bevorzugt werden als Basistensid in der Komponente (a) Citronensäureestermischungen von ethoxylierten Alkoholen der allgemeinen Formel (I) eingesetzt

R¹O(CH₂CH₂O)nH (I)

in der R¹ für einen linearen Alkylrest mit 4 bis 24 Kohlenstoffatomen und n für Zahlen von 5 bis 9 steht, mit der Maßgabe, dass in den Citronensäureestermischungen das Gewichtsverhältnis von Monoester : Diester im Bereich von 3: 1 bis 10 : 1 liegt.

Bei den erfindungsgemäß enthaltenen Citronensäureestermischungen der Komponente (b) handelt es sich um anionische Tenside, d.h. hauptsächlich um Verbindungen, die noch mindestens eine freie Carboxylgruppe enthalten. Dem entsprechend kann es sich um saure Ester oder deren Neutralisationsprodukte handeln. Vorzugsweise liegen die Partialester in Form der Alkali-, Erdalkali-, Ammonium-, Alkylammonium-, Alkanolammonium- und/oder Glucammoniumsalze vor.

Die Citronensäureestermischungen leiten sich von alkoxylierten Alkoholen ab, vorzugsweise von alkoxylierten aliphatischen Fettalkoholen mit 4 bis 24 Kohlenstoffatomen. Bevorzugt leiten sie sich ab von ethoxylierten Alkoholen mit 4 bis 24 Kohlenstoffatomen und insbesondere von solchen der allgemeinen Formel (II)

R²O(CH₂CH₂O)nH (II)

in der R² für einen linearen oder verzweigten Alkyl- und/oder Alkenylrest mit 4 bis 24 Kohlenstoffatomen und n für Zahlen von 1 bis 50 steht. Verbindungen der Formel (II) mit einem Ethoxylierungsgrad n von 1 bis 20 sind bevorzugt. Typische Beispiele sind die Addukte von durchschnittlich 1 bis 20, vorzugsweise 1 bis 10 und insbesondere 1 bis 8 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind Addukte von 1 bis 10 Mol Ethylenoxid an technische Fettalkohole mit 12 bis 18 Kohlenstoffatomen, wie beispielsweise Kokos-, Palm-, Palmkern- oder Talgfettalkohol. Eine besonders geeignete Fettalkoholmischung enthält 65 - 75 Gew.% C12-, 20 bis 30 Gew.% C14-, 0 - 5 Gew.% C16- und 0 bis 5 Gew.% C18- Alkohole. Diese Alkoholmischung ist kommerziell erhältlich, beispielsweise als Dehydol LS ™, ein Handelsprodukt der Cognis Deutschland GmbH & Co. KG. Eine weitere besonders geeignete Fettalkoholmischung enthält 45 -60 Gew.% C12-, 15 bis 30 Gew.% C14-, 5 - 15 Gew.% C16- und 8 bis 20 Gew.% C18- Alkohol. Diese Alkoholmischung ist ebenfalls kommerziell erhältlich, beispielsweise als Dehydol LT ™, ein Handelsprodukt der Cognis Deutschland GmbH & Co. KG.

### Komponente (b)

Die erfindungsgemäßen Formulierungen enthalten als Komponente (b) Öle und/oder Wachse, welche über haut- und haarpflegende Eigenschaften verfügen.

Als Komponente (b) dienen sowohl unpolare als auch polare Öle oder Mischungen hiervon. Hierzu zählen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃₋Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, I-sostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, O-leylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, E-rucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂₋Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂₋Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂₋Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane oder Silikonöle oder besonders bevorzugt Hydrogenated Polydecene.

Als Komponente (b) können jedoch auch feste Fette und/oder Wachse. Diese können auch in Mischung mit den im vorherigen Abschnitt genannten Ölen vorliegen. Typische Beispiele für Fette sind Glyceride, d.h. feste oder flüssige pflanzliche oder tierische Produkte, die im wesentlichen aus gemischten Glycerinestern höherer Fettsäuren bestehen. Hier sind insbesondere feste Mono- und Diglyceride zu nennen wie z.B. Glycerinmonooleat oder Glycerimnonostearat. Als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage. Tocopherole und ätherische Öle eignen sich ebenfalls als Komponente (b).

### Komponente (c)

Fakultativ können die erfindungsgemäßen gelförmigen Zubereitungen mono- oder polyfunktionelle Alkohole mit 1 bis 4 Kohlenstoffatomen in einer Menge von 0 bis 15 Gew.% bezogen auf die Gesamtformulierung enthalten. In einer bevorzugten Ausführungsform enthalten die erfindungsgemäßen Zubereitungen als Komponente (c) Glycerin, Ethylenglykol und/oder Propylenglykol.
Durch die Zugabe dieser Alkohole wird die Aufnahmefähigkeit der Zubereitungen für Öle erhöht. Außerdem kann der Brechungsindex der Wasserphase dem der dispergierten Ölphase angeglichen werden, so daß eine mögliche Trübung reduziert wird. Außerdem erhöht sich die Lagerstabilität der Gele bei niedrigen Temperaturen, z.B. bei -5°C.

### Wasserstoffperoxid

In einer weiteren bevorzugten Auführungsforrn weisen die erfindungsgemäßen Zubereitungen einen Gehalt an Wasserstoffperoxid auf. Die erfindungsgemäßen formstabilen Gele lassen sich einfach auf das Haar auftragen und verbleiben dort aufgrund ihrer hohen Formstabilität in engem Kontakt mit den Haaren ohne wie eine Flüssigkeit abzutropfen.
Hergestellt werden die erfindungsgemäßen Zubereitungen durch Verrühren mittels eines einfachen Rührwerks bei Temperaturen von ca. 70 °C. Dabei werden keine stark erhöhten Rührgeschwindigkeiten benötigt. Sollen Wachskomponenten eingearbeitet werden, müssen diese unbedingt vor Verrühren bis über ihren Schmelzpunkt erhitzt werden.

### Beispiele

Die in den folgenden Tabellen aufgeführten Beispiele geben die Inhaltsstoffe in Gew.% bezüglich der Aktivsubstanz an. Aufgeführt wird jedoch nicht nur der INCI-Name der Aktivsubstanz, sondern auch der Markenname eines entsprechenden Handelsproduktes.

**Tabelle 1: Öl-Gele**

| **INCI** Handelsname | **Konzentration [Gew. % Aktivsubstanz]** | | | | | | |
|---|---|---|---|---|---|---|---|
| **Glyceryl Oleate** Monomuls 90-018 | 8,00 | 6,88 | 8,00 | 8,00 | 8,00 | 8,00 | 8,00 |
| **Dicaprylyl Ether** Cetiol OE | 30,00 | 25,80 | 30,00 | 30,00 | - | | |
| **Paraffinöl** | - | - | - | - | 30,00 | 30,00 | 30,00 |
| **Laureth-7 Citrate** Plantapon LC7 | 15,00 | 12,90 | 15,00 | 15,00 | 15,00 | 15,00 | 11,50 |
| Lauryl Glucoside Plantacare 1200 UP | 8,40 | 7,23 | 7,06 | 5,04 | 5,04 | 7,56 | 8,57 |
| **1,2 Propylenglykol** | 3,00 | - | - | - | - | - | - |
| **NaCI** | - | 1,08 | - | - | - | 1,00 | - |
| **VE-Wasser** | 35,60 | 46,11 | 39,94 | 41,96 | 41,96 | 38,44 | 41,93 |
| **Aussehen** | leicht trübes Gel | leicht opake gelige Formul. | relativ klares gel | klares Gel | klares Gel | klares Gel | klares Gel |
| **pH-Wert (mit NaOH 15%ig)** | 4,40 | 4,34 | 3,75 | 3,73 | 5,22 | 3,70 | 4,68 |

**Tabelle 2: Öl-Gele**

| **INCI** Handelsname | **Konzentration [Gew. % Aktivsubstanz]** | | | |
|---|---|---|---|---|
| **Glyceryl Oleate** Monomuls 90-018 | 8,00 | 6,88 | 8,00 | 8,00 |
| **Dicaprylyl Ether** Cetiol OE | 30,00 | 25,80 | 30,00 | 30,00 |
| **Laureth-7 Citrate** Plantapon LC7 | 15,00 | 12,90 | 15,00 | 15,00 |
| **Sodium Lauryl Glucose Carboxylate (and) Lauryl Glucoside** Plantapon LGC SORB | 4,21 | - | - | - |
| **Disodium Cocoamphodiacetate** Dehyton DC | - | 7,17 | 7,00 | 5,00 |
| **1,2 Propylenglykol** | 3,00 | - | - | - |
| **NaCI** | - | 1,08 | - | - |
| **VE-Wasser** | 39,79 | 46,17 | 40,00 | 42,00 |
| | | | | |
| **Aussehen** | klar, gelig | opak, gelig | relativ klar, gelig | leicht opak, gelig |
| **pH-Wert (mit NaOH 15%ig)** | 4,05 | 4,06 | 4,16 | 3,88 |

**Tabelle 3 : Öl-Gele**

| **INCI** Handelsname | **Konzentration [Gew. % Aktivsubstanz]** | | | | | |
|---|---|---|---|---|---|---|
| **Glyceryl Oleate** Monomuls 90-018 | 8,00 | 8,00 | 8,00 | 8,00 | 8,00 | 8,00 |
| **Dicaprylyl Ether** Cetiol OE | 30,00 | 30,00 | 30,00 | 30,00 | 30,00 | 30,00 |
| **Laureth-7 Citrate** Plantapon LC7 | 15,00 | 15,00 | 15,00 | 15,00 | 15,00 | 15,00 |
| **Sodium Myreth Sulfate** Texapon K 14 spez. 70% | 11,66 | 11,66 | 11,66 | - | - | - |
| **Sodium Laureth Sulfate (and) Sodium Laureth-8 Sulfate (and) Magnesium Laureth Sulfate (and) Sodium Laureth-8 Sulfate (and) Sodium Oleth Sulfate (and) Magnesium Oleth Sulfate** Texapon ASV 50 | - | - | - | 8,33 | - | - |
| **Sodium Lauroyl Sarcosinate** Plantapon LS 30 | - | - | | | 4,76 | 4,76 |
| **1,2 Propylenglykol** | - | - | 3,00 | 3,00 | - | - |
| **NaCI** | 1,25 | - | - | - | - | 1,25 |
| **VE-Wasser** | 34,09 | 35,34 | 32,34 | 35,67 | 42,24 | 40,99 |
| **Aussehen** | relatives trübes Gel | klares Gel | klares Gel | relativ trübes Gel | relativ klares Gel | relativ trübes Gel |
| **pH-Wert (mit NaOH 15%ig)** | 5,12 | 3,87 | 4,30 | 4,00 | 5,16 | 4,50 |

**Tabelle 4: Wasserstoffperoxidhaltiges Gel**

| **INCI** Handelsname | **Konzentration [Gew. % Aktivsubstanz]** |
|---|---|
| **Glyceryl Oleate** Monomuls 90-018 | 8,00 |
| **Dicaprylyl Ether** Cetiol OE | 30,00 |
| **Laureth-7 Citrate** Plantapon LC7 | 15,00 |
| **Lauryl Glucoside** Plantacare 1200 UP | 7,06 |
| **Wasserstoffperoxid** | 3,00 |
| **VE-Wasser** | 36,94 |
| **Aussehen** | klar, gelig |

## Patentansprüche

1. Gelförmige kosmetische Zubereitung enthaltend
(a) 5 bis 50 Gew.% Tenside,
(b) 5 bis 50 Gew.% Öle und/oder Wachse,
(c) 0 bis 15 Gew.% wasserlösliche Polyole,
wobei die Komponente (a) mindestens 10 Gew.% in bezug auf die Gesamtzubereitung eines Citronensäureesters alkoxylierter Alkohole enthält und die Summe der Komponenten (a) und (b) in der Gesamtzubereitung 10 bis 70 Gew.% ausmacht.

2. Zubereitung gemäß Anspruch 1, **dadurch gekennzeichnet, daß** die Komponente (a) neben Citronensäureestern alkoxylierter Alkohole Cotenside enthält, die ausgewählt sind aus der Gruppe, die gebildet wird von anionischen, kationischen, nichtionischen, amphoteren und/oder zwitterionischen Tensiden.

3. Zubereitung gemäß wenigstens einem der Ansprüche 1 und/oder 2, **dadurch gekennzeichnet, daß** die Komponente (a) neben Citronensäureestern alkoxylierter Alkohole Cotenside enthält, die ausgewählt sind aus der Gruppe, die gebildet wird von Betainen, acylierten Aminosäuren, Alkylethersulfaten, Alkylsulfaten und Alkyloligoglykoside.

4. Zubereitungen gemäß wenigstens einem der Ansprüche 1 bis 3, **dadurch gekennzeichnet, daß** als Komponente (a) Citronensäureestermischungen von ethoxylierten Alkoholen der allgemeinen Formel (I) eingesetzt werden
R¹O(CH₂CH₂O)nH (I)
in der R¹ für einen linearen Alkylrest mit 4 bis 24 Kohlenstoffatomen und n für Zahlen von 5 bis 9 steht, mit der Maßgabe, dass in den Citronensäureestermischungen das Gewichtsverhältnis von Monoester : Diester im Bereich von 3: 1 bis 10 : 1 liegt.

5. Zubereitung gemäß wenigstens einem der Ansprüche 1 bis 4, **dadurch gekennzeichnet, daß** als Komponente (c) Glycerin, Ethylenglykol und/oder Propylenglykol eingesetzt wird.

6. Zubereitung gemäß wenigstens einem der Ansprüche 1 bis 5, **dadurch gekennzeichnet, daß** die Zubereitung weiterhin einen Gehalt an Wasserstoffperoxid aufweist.
